# EUROPÄISCHE PATENTSCHRIFT

(11) **EP 1 697 004 B1**
(45) Veröffentlichungstag und Bekanntmachung des Hinweises auf die Patenterteilung: **04.09.2013**
(21) Anmeldenummer: 04818131.7
(22) Anmeldetag: 08.11.2004
(51) Int. Cl.: A61N 5/10

(54) **PATIENTENIDENTIFIKATIONSSYSTEM UND PATIENTENPOSITIONIERUNGSVERFAHREN**
PATIENT IDENTIFICATION SYSTEM AND PATIENT POSITIONING METHOD
SYSTÈME D'IDENTIFICATION DE PATIENTS ET PROCÉDÉ DE POSITIONNEMENT DE PATIENTS

(30) Priorität: 08.11.2003 DE 10352556
(43) Veröffentlichungstag der Anmeldung: 06.09.2006
(73) Patentinhaber: Medical Intelligence Medizin-Technik GmbH, 86830 Schwabmünchen (DE)
(72) Erfinder: HIERONIMI, Christian, 86836 Graben (DE); VOGELE, Michael, Dr., 86830 Schwabmünchen (DE)
(74) Vertreter: Downing, Michael Philip
(86) Internationale Anmeldenummer: PCT/EP2004/012625
(87) Internationale Veröffentlichungsnummer: WO 2005/044378

(56) Entgegenhaltungen:
- EP-A- 0 510 615
- GB-A- 2 340 716
- US-A- 5 820 553
- US-B1- 6 445 300

## Beschreibung

Die Erfindung betrifft ein Patientenidentifikationssystem, mit dem ein Patient digital erkennbar und lokalisierbar ist, und weiterhin ein Patientenpositionierungsverfahren, bei dem ein Patient mit Hilfe digitaler Erkennung und Lokalisierung in einer Sollposition positioniert wird.

Die wiederholte Positionierung eines Patienten in einer bestimmten, vorgegebenen Position im Raum ist bei medizinischen Therapie - und / oder Diagnoseverfahren von großer Bedeutung. Beispielhaft sei die Strahlentherapie angeführt. Die Strahlentherapie wird als vorbereitende, begleitende oder anschließende Therapie bei gut - und bösartigen Tumoren verwendet. Bei der Bestrahlung eines Patienten ist dessen Position und Lage zur Strahlenquelle äußerst wichtig. Die Strahlung soll möglichst präzise auf die zu behandelnde Stelle, das sogenannte Iso-Zentrum, gerichtet werden. Zum einen soll der Tumor möglichst vollständig von der Strahlung getroffen werden, zum anderen soll jedoch benachbartes, gesundes Gewebe möglichst nicht von der Strahlung getroffen werden. Folglich muss das Iso-Zentrum möglichst exakt zur Strahlenquelle ausgerichtet werden. Da in aller Regel eine einzige Bestrahlung nicht ausreichend ist, ist es erforderlich in regelmäßigen Zeitabständen den Patienten immer wieder erneut zur Strahlenquelle auszurichten. Dieses (Re-) Positionieren eines Patienten geschieht bis jetzt üblicherweise durch entsprechend geschultes Personal.

Nachteilig an der Positionierung durch Personal ist zum einen, dass die Qualität der Positionierung, dass heißt die Genauigkeit, von einer Person abhängig ist und dementsprechend Schwankungen unterliegt. Weiterhin sollte die Positionierung stets durch dieselbe Person erfolgen, um die Schwankungen auf diejenigen zu beschränken, die innerhalb einer Person vorliegen und nicht zusätzlich Ungenauigkeiten dadurch einfließen, dass verschiedene Personen die Positionierung zu verschiedenen Zeitpunkten vornehmen. Schließlich ist nicht nur die schwankende Genauigkeit der Patientenpositionierung problematisch, sondern auch der Zeitbedarf. Es ist davon auszugehen, dass eine Person in der Regel einen erhöhten Zeitbedarf im Vergleich zu Maschinen aufweisen wird. Die automatische Positionierung ist Jedoch bis jetzt nur stark eingeschränkt möglich. Voraussetzung hierfür ist, dass der Patient stets identisch auf die Patientenliege vor der Positionierung gelagert wird, denn nur dann kann eine automatisch einstellbare Liege stets in dieselbe Position fahren. Damit wären Liegen in der Art von Hebebühnen einsetzbar, wie sie beispielsweise in der automatischen Fertigung verwendet werden. Sobald jedoch der Patient nicht exakt identisch abgelegt ist, wird eine automatische Positionierung unmöglich. Infolgedessen besteht ein hohes Risiko, dass gesundes Gewebe behandelt wird und erkranktes Gewebe ausgespart wird.

Wichtig für eine automatische Positionierung ist auch, dass das Positionierungssystem erkennen kann, um welchen Patienten es sich handelt. Da bei einer automatischen Positionierung eine "Sichtkontrolle" durch einen radiologisch-technischen Assistenten entfällt, muss sicher gestellt sein, dass auch der richtige Patient mit der richtigen Position, die von dem Positionierungssystem angefahren wird, verknüpft wird, also keine Behandlung an einem "falschen" Patienten erfolgt.

Die US 5820553 offenbart ein System und Verfahren zur Identifizierung und Abfertigung Individueller Patienten unter einer Reihe von Patienten, einschließlich Speicherung von Positionsdaten, die für patientenspezifische Anordnungen von Targets repräsentativ sind, die an den Patienten zur Ausrichtung eines therapeutischen Nutzstrahlenbündels angebracht sind.

Die US 6445300 offenbart eine Identifizierungskarte, die ein kleines Funksendegerät umfasst, wobei die Karte wichtige persönliche Informationen wie z. B, Name des Benutzers, Adresse und aktuelle Medikationen enthält.

Die GB 2340716 offenbart ein Überwachungssystem für Patientenpositionierung, bei dem ein Satz flacher farbiger Scheiben am Patienten befestigt und durch Triangulation abgebildet wird.

Aufgabe der folgenden Erfindung ist es daher, ein Patientenidentifikationssystem bereitzustellen mit dem ein Patient erkennbar und im Raum lokalislerbar ist. Weiterhin ist Aufgabe, ein entsprechendes Patientenpositionierungsverfahren bereitzustellen.

Diese Aufgabe wird gelöst durch ein Patientenidentifikationssystem nach Anspruch 1 und ein Patientenpositionierungsverfahren nach Anspruch 8. Bevorzugte Ausführungsbeispiele sind Gegenstand der Unteransprüche.

Das erfindungsgemässe Patientenidentifikationssystem weist zum einen Mittel zur Kennzeichnung des Patienten auf, und zum anderen Mittel zum Lokalisieren des Patienten im Behandunqsnaum der Form von zumindest einem Marker, bevorzugt drei Markern.

Diese Mittel sind digital erfassbar. Das erfindungsgemässe System ist auf mindestens einem Träger angeordnet und dieser Träger ist mittelbar und/oder unmittelbar am Patienten befestigt. Anders ausgedrückt ist ein Träger vorgesehen, auf dem sowohl Mittel zur Kennzeichnung des Patienten als auch Mittel zum Lokalisieren des Patienten im Raum angeordnet sind, so dass mit diesem auf dem Träger angeordneten System ein Patient digital erkennbar ist, um welchen Patienten es sich handelt und wie der Patient im Behandlungsraum lokalisiert ist, in dem Sinne, dass die Position des Patienten in dem Raum exakt bekannt ist. Genauer ausgedrückt ist bekannt, wo sich der mittelbar oder unmittelbar am Patienten befestigte Träger oder die auf dem Träger angeordneten Marker im Raum befinden. Aufgrund der Befestigung am Patienten kann dann geschlossen werden, wo sich das Iso-Zentrum des jeweiligen Patienten im dreidimensionalen Raum befindet.

Bei dem Kennzeichnungsmittel handelt es sich um ein RFID-Funk-Etikett (RFID-Tag). Wichtig ist, dass das Mittel digital erfassbar ist. Zusätzlich ist vorgesehen, dass der Träger auch für das Personal unmittelbar lesbare Information über den Patienten enthält, beispielsweise indem der Name und die Patientennummer auf dem Träger angebracht sind. Der Träger hat vorzugsweise die Form einer Karte, in der Art einer bekannten Bankkarte. Diese Karte kann an einer Struktur zur Fixierung des Patienten oder direkt an diesem befestigt sein. Derartige Strukturen sind beispielsweise Kopfhalterungen, Mundhalterungen, ein stereotaktischer Rahmen, oder Körperfixiermittel. Alternativ kann der erfindungsgemäße Träger auch die Struktur zur Fixierung des Patienten selbst sein. Dies bedeutet, dass die beiden Mittel unmittelbar auf dieser Struktur vorgesehen sind.

Vorteil des erfindungsgemäßen Patientenidentifikationssystems ist, dass damit ein Patient sowohl digital erfassbar ist, sprich identifizierbar ist, als auch seine Position im Raum digital erfassbar ist. Dadurch werden die Grundvoraussetzungen für ein automatisches Positionierungsverfahren geschaffen, insbesondere auch eine zuverlässige Dokumentation erzielt. Ein hierfür eingesetzter Roboter oder ein anderes System kann folglich Identität und Lage des Patienten erfassen und mit Hilfe der daraus gewonnenen Information den Patienten automatisch positionieren. Durch die Identifikationsdaten erkennt das automatische System, um welchen Patienten es sich handelt und kann so die für den Patienten vorhandenen Daten aufrufen. Bei diesen Daten sind auch vorzugsweise Daten dabei, die Auskunft darüber geben, wo sich das zu behandelnde Iso-Zentrum des Patienten befindet. Falls diese Daten nicht übereinstimmen, wird das Therapiegerät, z.B. ein Linearbeschleuniger gesperrt bzw. sein Betrieb nicht freigegeben. Ferner stellt dieses Patientenidentifizierungssystem die Information darüber bereit, wie und wo der Patient sich im Raum befindet. Zwar wird versucht, einen Patienten stets gleichbleibend auf der Behandlungsliege zu lagern, dies ist jedoch nicht immer exakt gleichbleibend möglich. Gerade Abweichungen, die durch eine geringfügige Rotation des Patienten um die Längsachse erzeugt werden, sind relativ schwierig mit Hilfe der klassischen Lagerungsmöglichkeiten zu erfassen. Mit Hilfe des Mittels zum Lokalisieren des Patienten des erfindungsgemäßen Patientenidentifikationssystems sind jedoch genau diese Daten bekannt. Die aktuelle Position des Patienten im Raum ist dadurch bekannt und ein Positionierungssystem kann die daraus gewonnenen Daten verarbeiten, und mit den bekannten Referenzdaten des Iso-Zentrums verknüpfen und so entsprechend die Positionierung des Patienten vornehmen. Dadurch ist gewährleistet, dass der Patient stets exakt zum Behandlungsgerät ausgerichtet ist. Wie oben ausgeführt, ist die exakte Positionierung des Patienten dabei unabdingbar, insbesondere in der Strahlentherapie als auch bei durch Operationsroboter vorgenommenen chirurgischen Eingriffen.

Das erfindungsgemäße Patientenpositionierungsverfahren weist folgende Schritte auf:
a) Fixierung eines Patienten auf einer Liege,
b) Anbringen des Patientenidentifikationssystems an dem Patienten,
c) Verbringen des Patienten in eine Sollposition,
d) Erkennen des Patienten,
e) Anfertigen einer bevorzugt digitalen Aufnahme einer Sollposition unter Zuhilfenahme des Patientenidentifikationssystems,
f) bei erneuerter Positionierung dieses Patienten, Wiederholen der Schritte a-c
g) Anfertigen einer bevorzugt digitalen Aufnahme der Istposition des Patienten mit Hilfe des Patientenidentifikationssystems,
h) Vergleichen der Aufnahmen der Istposition
i) bei festgestellter Abweichung der Ist- und der Sollposition, Verstellen der Liege bis die Istposition im Wesentlichen mit der Sollposition übereinstimmt.

Wie ausgeführt, wird mit Hilfe des Patientenidentifikationssystems der Patient automatisch erkannt und seine Position im Raum automatisch bestimmt. Dadurch kann die automatische Positionierung des Patienten erfolgen. Alternativ dazu, können die gewonnenen Daten auch verwendet werden, um beispielsweise auf einem Bildschirm die Daten anzuzeigen, so dass das Personal, beispielsweise ein radiologisch - technischer Assistent, die Positionierung des Patienten unter Zuhilfenahme der gewonnenen Daten manuell vornehmen kann.

Vorzugsweise wird bei der automatischen Positionierung des Patienten das Verstellen der Patientenliege durch zumindest einen Hexapoden erfolgen. Ein Vorteil bei der Einstellung der Liege mit dem Hexapoden ist, dass dadurch optimal sämtliche sechs Freiheitsgrade angefahren werden können. Somit ist die Position des Patienten nicht nur mit Hilfe der X-, Y- und Z- Achsen einstellbar, sondern auch über die drei rotatorischen Achsen. Die digitalen Aufnahmen können mit zumindest einer CCD - oder Infrarot-Kamera oder einem Funkempfänger zum Orten der Positionsdaten erfolgen. Weiterhin können die Aufnahmen mit Hilfe der aktiven Triangulation, dem Lichtschnittverfahren und/oder dem kodierten Lichtansatz erfolgen. Dadurch sind besonderst exakte Aufnahmen möglich, was wichtig ist, um die Patientendaten möglichst exakt zu erfassen. Weiterhin können nicht nur die Marker des Patientenidentifikationssystems erfasst werden und Auskunft über die Position des Patienten geben, sondern es können auch Landmarken des Patienten selber verwendet werden, beispielsweise Knochenmerkmale oder markante Punkte der Gesichtszüge, beispielsweise Nase, Kinn oder Augenhöhlen. Dadurch werden die durch das erfindungsgemäße Patientenidentifikationssystem bereitgestellten Daten erweitert und optimiert. So wird die exakte Positionierung des Patienten weiter verbessert, beispielsweise die exakte Positionierung eines Mundstücks überprüft.

Nachfolgend wird die Erfindung an Hand der Zeichnungen näher erläutert und beschrieben. Es zeigen:
- Fig. 1: ein Patientenidentifikationssystem; und
- Fig. 2: ein weiteres Patientenidentifikationssystem; und
- Fig. 3: das Patientenidentifikationssystem in Anwendung.

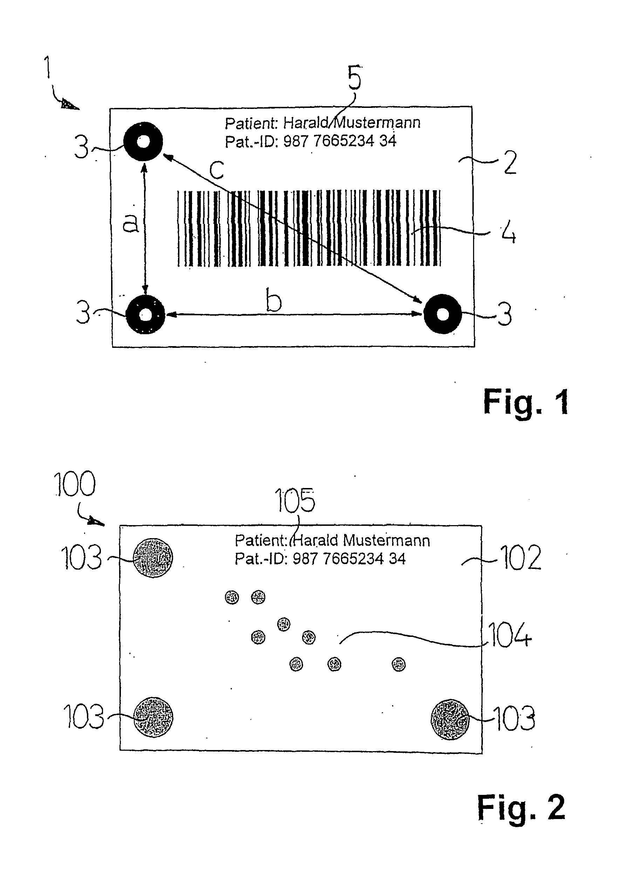

Das Patientenidentifikationssystem 1 der Fig. 1 weist einen Träger 2 auf, der die Form einer Karte aufweist. Auf dem Träger 2 sind drei Marker 3 angeordnet, beispielsweise aufgedruckte schwarze Ringe. Die geometrische Anordnung der Ringe auf dem Träger 2 zeigt hier lediglich ein Ausführungsbeispiel. Wichtig ist dabei, dass die Abstände a, b, c zwischen den einzelnen Markern 3 möglichst groß sein sollten, weshalb sie bevorzugt in den Ecken des Trägers 2 angeordnet werden. Wichtig ist weiter, dass keiner der drei Abstände a, b, c gleich groß sein sollte, um ein möglichst genaues 3D - Koordinatensystem zu erhalten, an dem sich der Computer des Positionierungssystems bei den Berechnungen orientieren kann. Die drei Marker 3 werden digital erfasst. Aus ihrer Position im Raum und aus der bekannten Position der Aufnahmekameras kann dann die Position des Patienten im Raum berechnet werden.

Auf dem Träger 2 ist weiter ein Mittel zur Kennzeichnung des Patienten angeordnet, genauer ein Barcode, das Kein Ausführunqsbeispiel der Erfindung ist. Barcodes, auch bekannt unter Strichcode, sind gängige Mittel, um Information digital erfassbar zu machen. Aus der Strichstärke und den Abständen der einzelnen Striche zueinander ergibt sich die weiterzugebende Information. Schließlich kann auf dem Träger 2 auch lesbar die Identität des Patienten angegeben werden, z. B. mit Hilfe des aufgedruckten Namens oder andere Informationen, beispielsweise einem Bild oder einer Patienten-ID-Nummer. Die in dem Patientenidentifikationssystem 1 gewählte Wiedergabeform des Mittels zur Kennzeichnung des Patienten und des Mittels zur Lokalisierung des Patienten sind mit Hilfe einer CCD-Kamera erfassbar. Das in Fig. 2 dargestellte Patientenidentifikationssystem 100 ist dagegen mit einer Infrarotkamera digital erfassbar. Die Marker 103 ebenso wie das Mittel zur Kennzeichnung 104 das kein Ausführungsbeispiel der Erfindung ist, sind retroreflektierend, so dass das von der Kamera oder einem Leuchtmittel ausgesandte Licht zurückgeworfen wird, wodurch es für die Infrarotkamera erfassbar wird. Bei den Markern 103 handelt es sich beispielsweise um retroreflektierende Punkte. Die exakte geometrische Form und Struktur der Marker 3, 103 ist dabei unerheblich. Es kann sich dabei um jede geeignete Struktur handeln, beispielsweise um Punkte, Ringe oder Vielecke. Das Mittel zur Kennzeichnung des Patienten 104, das kein Ausführungsbeispiel der Erfindung ist im Figur 2 ein Muster aus retroreflektierenden Markierungen, beispielsweise Punkten. Durch eine Anzahl an retroreflektierenden Punkten kann ein geometrisches Muster angeordnet werden, anhand dessen auf die Identität des Patienten zurückgeschlossen werden kann. Das Muster ist digital erfassbar.

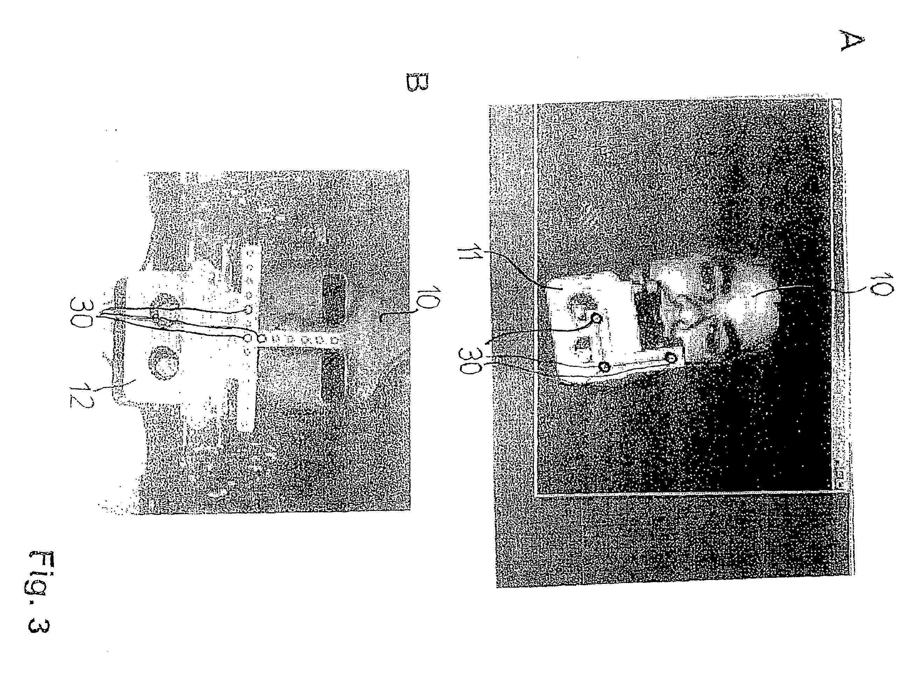

Im Figur 3 ist eine abgewandelte Ausführung eines Patientenidentifikationssystems dargestellt. Bei dem Träger, auf dem das System angeordnet ist, handelt es sich um Strukturen zur Fixierung des Patienten. In Figur 3A ist dies eine Mundhalterung 11 und in Figur 3B eine Kopfhalterung 12. Diese Strukturen sind jeweils am Patienten 10 fixiert. Über sie wird der Patient wiederum auf der Liege fixiert. Auf der Mundhalterung 11 bzw. der Kopfhalterung 12 sind jeweils drei Marker 30 räumlich angeordnet, so dass aus der geometrischen Anordnung der drei Marker 30 auch hier die exakte Positionierung des Patienten berechnet werden kann. Dies kann auch mit einem Funk-Etikett (RFID-Tag mit integrierter Flachantenne) erfolgen, das beispielsweise auf der Mundhalterung 11 aufgeklebt ist. Mit einem nicht dargestellten Funkempfänger (z. B. mit einem WLAN-Sende- und Empfangsprotokoll) lassen sich die Positionsdaten und die Ausrichtung des Patienten dreidimensional erfassen, aber auch seine individuellen Identifikationsdaten. Derartige Funk-Etiketten lassen sich auch auf dem Patienten direkt befestigen, z.B. in der Nähe der zu behandelnden Stelle aufkleben.

## Patentansprüche

1. Patientenidentifikationssystem (1, 100), mit dem ein Patient erkennbar und lokalisierbar im Behandlungsraum ist, wobei dieses System aufweist : a) Mittel zur Kennzeichnung des Patienten (4,104), b) Mittel zum Lokalisieren des Patienten im Behandlungsraum in der Form von zumindest einem Marker (3,30, 103), wobei diese Mittel jeweils digital erfassbar sind, und wobei das System auf zumindest einem Träger (2, 11, 12,102) angeordnet ist und der Träger mittelbar oder unmittelbar am Patienten (10) befestig bar ist, das **dadurch gekennzeichnet, dass** Mittel zur Kennzeichnung des Patienten ein RFID-Funk-Etikett ist.

2. Patientenidentifikationssystem nach Anspruch 1, **dadurch gekennzeichnet, dass** die Marker (3,30, 103) retroreflektierende oder farbige geometrische Strukturen, insbesondere Punkte, Ringe oder Vielecke sind.

3. Patientenidentifikationssystem nach einem der vorangegangenen Ansprüche, **dadurch gekennzeichnet, dass** die Mittel zur Kennzeichnung mit einem Funkempfänger erfassbar sind.

4. Patientenidentifikationssystem nach einem der vorangegangenen Ansprüche, **dadurch gekennzeichnet, dass** die Mittel zum Lokalisieren mit einem Funkempfänger oder einer CCD-und/oder Infrarot-Kamera digital erfassbar sind.

5. Patientenidentifikationssystem nach einem der vorangegangenen Ansprüche, **dadurch gekennzeichnet, dass** der Träger eine Karte ist.

6. Patientenidentifikationssystem nach Anspruch 5, **dadurch gekennzeichnet, dass** die Karte an einer Struktur zur Fixierung des Patienten, insbesondere einer Kopfhalterung (12), einen Körperfixiermittel, einer Mundhalterung (11) oder einem stereotaktischen Rahmen befestigt ist.

7. Patientenidentifikationssystem nach einem der Ansprüche 1 bis 4, **dadurch gekennzeichnet, dass** der Träger einer Struktur zur Fixierung des Patienten, insbesondere eine Kopfhalterung (12), ein Körperfixiermittel, eine Mundhalterung (11) oder ein stereotaktischer Rahmen ist.

8. Patientenpositionierungsverfahren, aufweisend die folgenden Schritte : a) Fixierung eines Patienten (10) auf einer Liege, b) Anbringen des Patientenidentifikationssystems (1, 100) nach Anspruch 1 an dem Patienten, c) Erkennen des Patienten, d) Verbringen des Patienten in eine Sollposition, e) Anfertigen einer Aufnahme der Sollposition unter Zuhilfenahme des Patientenidentifikationssystems, f) bei Repositionierung dieses Patienten, Wiederholen der Schritte a bis c, g) Anfertigen einer Aufnahme der Istposition des Patienten mit Hilfe des Patientenidentifikationssystems, h) Vergleichen der Aufnahmen der Istposition und der Sollposition, und i) bei festgestellter Abweichung der Istposition von der Sollposition, Verstellen der Liege bis die Istposition im Wesentlichen mit der Sollposition übereinstimmt.

9. Patientenpositionierungsverfahren nach Anspruch 8, **dadurch gekennzeichnet, dass** die Schritte d und i automatisch und/oder manuell erfolgen.

10. Patientenpositionierungsverfahren nach Anspruch 8 oder 9, **dadurch gekennzeichnet, dass** die Schritte d und/oder i durch einen Hexapoden erfolgt.

11. Patientenpositionierungsverfahren nach einem der Ansprüche 8 bis 10, **dadurch gekennzeichnet, dass** das Anfertigen der Aufnahme mit zumindest einem Funkempfänger oder einer CCD-und/oder Infrarot-Kamera erfolgt.

12. Patientenpositionierungsverfahren nach einem der Anspruch 8 bis 11, **dadurch gekennzeichnet, dass** das Anfertigen der Aufnahmen mit Hilfe der aktiven Triangulation, dem Lichtschnittverfahren und/oder dem kodierten Lichtansatz erfolgt.

## Claims

1. Patient identification system to which a patient is identified and localized, said system comprising: a) means for the identification of the patient, b) means for locating the patient in the treatment room in the form of at least one marker, said means are digitally detected and the system is arranged on at least one carrier and the carrier is attached indirectly or directly to the patient, **characterised in that** the means for the identification of the patient is an RFID radio label.

2. Patient identification system according to claim 1 **characterised in that** the markers (3, 30, 103) are retro-reflective or coloured geometric structures, such as dots, rings or polygons.

3. Patient identification system according to any one of the preceding claims, **characterised in that** the means for identification are detected via a radio receiver.

4. Patient identification system according to any one of the preceding claims, **characterised in that** the means for locating can be digitally detected with a radio receiver or a CCD and/or infrared camera.

5. Patient identification system according to any one of the preceding claims, **characterised in that** the carrier is a card.

6. Patient identification system according to claim 5 **characterised in that** the card is fixed to a structure for the fixing of the patient, in particular a head restraint (12), a means of body fixing, a mouth restraint (11) or a stereotactic frame.

7. Patient identification system according to any one of claims 1 to 4, **characterized in that** the carrier is a structure for the fixing of the patient, in particular a head restraint (12), a means of body fixing, a mouth restraint (11) or a stereotactic frame.

8. Patient positioning method comprising the steps of: a) fixing a patient (10) on a treatment table, b) attaching the patient identification system (1, 100) according to claim 1 to the patient, c) identifying the patient, d) placing the patient in a desired position e) making a record of the desired position with the aid of the patient identification system, f) repetition of steps a to c, on repositioning the patient, g) making a record of the actual position of the patient with the patient identification system, h) comparing the recording of the actual position and the desired position, and i) on establishing the deviation of the actual position from the selected position, adjusting the treatment table until the actual position is essentially the same as the desired position.

9. Patient positioning method according to claim 8, **characterised in that** steps d and i take place automatically and/or manually.

10. Patient positioning method according to claim 8, **characterised in that** steps d and/or i take place using a hexapod.

11. Patient positioning method according to any one of claims 8 to 10, **characterised in that** the record is made using at least one of a radio receiver or a CCD and/or infrared camera.

12. Patient positioning method according to any one of claims 8 to 11, **characterised in that** the record is made by means of active triangulation, the optical section method, and/or the coded light approach.

## Revendications

1. Système d'identification de patient (1, 100), avec lequel un patient est identifiable et localisable dans la salle d'intervention, dans lequel ce système présente : a) des moyens de caractérisation du patient (4, 104), b) des moyens de localisation du patient dans la salle d'intervention sous la forme d'au moins un marqueur (3, 30, 103), dans lequel ces moyens sont respectivement détectables de manière numérique, et dans lequel le système est disposé sur au moins un support (2, 11, 12, 102) et le support peut être fixé directement ou indirectement sur le patient (10), **caractérisé en ce que** le moyen de caractérisation du patient est une radio-étiquete RFID.

2. Système d'identification de patient selon la revendication 1, **caractérisé en ce que** les marqueurs (3, 30, 103) sont des structures rétroréfléchissantes ou géométriques colorées, en particulier des points, des anneaux ou des polygones.

3. Système d'identification de patient selon l'une des revendications précédentes, **caractérisé en ce que** les moyens de caractérisation du patient sont détectables avec un récepteur radio.

4. Système d'identification de patient selon l'une des revendications précédentes, **caractérisé en ce que** les moyens de localisation sont détectables de manière numérique avec un récepteur radio ou une caméra CCD et/ou infrarouge.

5. Système d'identification de patient selon l'une des revendications précédentes, **caractérisé en ce que** le support est une carte.

6. Système d'identification de patient selon la revendication 5, **caractérisé en ce que** la carte est fixée à une structure pour la fixation du patient, en particulier un appui-tête (12), un moyen de fixation du corps, un support buccal (11) ou un cadre stéréotaxique.

7. Système d'identification de patient selon l'une des revendications 1 à 4, **caractérisé en ce que** le support est une structure de fixation du patient, en particulier un appui-tête (12), un moyen de fixation du corps, un support buccal (11) ou un cadre stéréotaxique.

8. Procédé de positionnement de patient, présentant les étapes suivantes : a) fixation d'un patient (10) sur une couchette, b) mise en place du système d'identification de patient (1, 100) selon la revendication 1 sur le patient, c) reconnaissance du patient, d) mise du patient dans une position théorique, e) exécution d'un enregistrement de la position théorique à l'aide du système d'identification de patient, f) lors du repositionnement de ce patient, répétition des étapes a à c, g) exécution d'un enregistrement de la position réelle à l'aide du système d'identification de patient, h) comparaison des enregistrements de la position réelle et de la position théorique, et i) lors de la constatation d'un écart entre la position réelle et la position théorique, réglage de la couchette jusqu'à ce que la position réelle concorde pour l'essentiel avec la position théorique.

9. Procédé de positionnement de patient selon la revendication 8, **caractérisé en ce que** les étapes d et i s'effectuent automatiquement et/ou manuellement.

10. Procédé de positionnement de patient selon la revendication 8 ou 9, **caractérisé en ce que** les étapes d et/ou i s'effectuent au moyen d'un hexapode.

11. Procédé de positionnement de patient selon l'une des revendications 8 à 10, **caractérisé en ce que** l'exécution de l'enregistrement s'effectue avec au moins un récepteur radio ou une caméra CCD et/ou infrarouge.

12. Procédé de positionnement de patient selon l'une des revendications 8 à 11, **caractérisé en ce que** l'exécution des enregistrements s'effectue au moyen de la triangulation active, de la méthode de la coupe optique et/ou de l'approche par lumière codée.
